(19) Europäisches Patentamt
European Patent Office
Office européen des brevets

(11) EP 3 449 825 A1

(12) **EUROPEAN PATENT APPLICATION**
published in accordance with Art. 153(4) EPC

(43) Date of publication:
06.03.2019 Bulletin 2019/10

(51) Int Cl.:
*A61B 5/1455* (2006.01)    *A61B 5/00* (2006.01)

(21) Application number: 17789404.5

(86) International application number:
PCT/JP2017/015908

(22) Date of filing: 20.04.2017

(87) International publication number:
WO 2017/188121 (02.11.2017 Gazette 2017/44)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: 27.04.2016 JP 2016089910

(71) Applicant: **Asahi Kasei Pharma Corporation**
**Chiyoda-ku**
**Tokyo 1000006 (JP)**

(72) Inventors:
• **YAMAMOTO Kiyoshi**
  **Tokyo 100-0006 (JP)**
• **NOGUCHI Yoshihiro**
  **Tokyo 100-0006 (JP)**

(74) Representative: **Hoffmann Eitle**
**Patent- und Rechtsanwälte PartmbB**
**Arabellastraße 30**
**81925 München (DE)**

(54) **ESTIMATION DEVICE**

(57) An estimating device to estimate blood oxygen concentration of a living body including: an acquiring unit to acquire optical information from the living body; a converting unit to convert a signal included in the optical information into a first color-difference signal; and a calculating unit to calculate the blood oxygen concentration of the living body based on the first color-difference signal is provided. Also, the acquiring unit may acquire optical information based on reflected light from the living body. Furthermore, the converting unit may convert a signal included in the optical information into a second color-difference signal that is different from the first color-difference signal; and the calculating unit may calculate the blood oxygen concentration based on the first color-difference signal and the second color-difference signal.

FIG. 1

EP 3 449 825 A1

## Description

### BACKGROUND

#### 1. TECHNICAL FIELD

The present invention relates to an estimating device.

#### 2. RELATED ART

Conventionally, estimating devices to estimate blood oxygen concentration of a living body from RGB signals have been known (refer to Non-Patent Document 1, Patent documents 1-4, for example).

[Non-Patent Document]

Non-Patent Document 1: Ufuk Bal, "Non-contact estimation of heart rate and oxygen saturation using ambient light", USA, The Optical Society of America, Biomedical Optics Express 86, January 1, 2015, Vol. 6, Issue 1, pp. 86-97

[Patent document]

Patent document 1: Japanese Patent Application, Publication No. 2012-143399
Patent document 2: Japanese Patent Application, Publication No. 2012-125501
Patent document 3: Japanese Patent Application, Publication No. H6-285050
Patent document 4: Japanese Translation of PCT International Patent Application No. 2014-529439

However, in order to estimate the blood oxygen concentration from RGB signals, the conventional estimating devices detect the peak and bottom of the pulse wave.

[General Disclosure]

The first aspect of the present invention provides an estimating device to estimate blood oxygen concentration of a living body, including: an acquiring unit to acquire optical information from the living body; a converting unit to convert a signal included in the optical information into a first color-difference signal; and a calculating unit to calculate the blood oxygen concentration of the living body based on the first color-difference signal.

The summary clause does not necessarily describe all necessary features of the embodiments of the present invention. The present invention may also be a sub-combination of the features described above.

### BRIEF DESCRIPTION OF THE DRAWINGS

Fig. 1 shows an outline of a configuration of an estimating device 100.
Fig. 2 shows an outline of a configuration of an estimating system 200 according to Example 1.
Fig. 3 is an exemplary flowchart illustrating operations of the estimating device 100 according to Example 1.
Fig. 4 shows an exemplary outline of an estimating method of the estimating device 100 according to Example 1.
Fig. 5 shows exemplary RGB signals acquired by the acquiring unit 20.
Fig. 6 shows exemplary YCbCr signals acquired by the acquiring unit 20.
Fig. 7 shows an actual value of $SpO_2$ that is detected from the living body 110.
Fig. 8 shows an estimated value of the blood oxygen concentration that is estimated by the estimating device 100.
Fig. 9 shows an exemplary configuration of the estimating device 100 according to Example 2.
Fig. 10 shows an exemplary configuration of the estimating device 100 according to Example 2.
Fig. 11 shows exemplary wavelengths of irradiation lights when the lights emitted from the light source 10 include wavelengths of visible light.
Fig. 12 shows exemplary wavelengths of the irradiation lights when the lights emitted from the light source 10 include wavelengths of other than visible light.

### DESCRIPTION OF EXEMPLARY EMBODIMENTS

Hereinafter, (some) embodiment(s) of the present invention will be described. The embodiment(s) do(es) not limit the invention according to the claims, and all the combinations of the features described in the embodiment(s) are not necessarily essential to means provided by aspects of the invention.

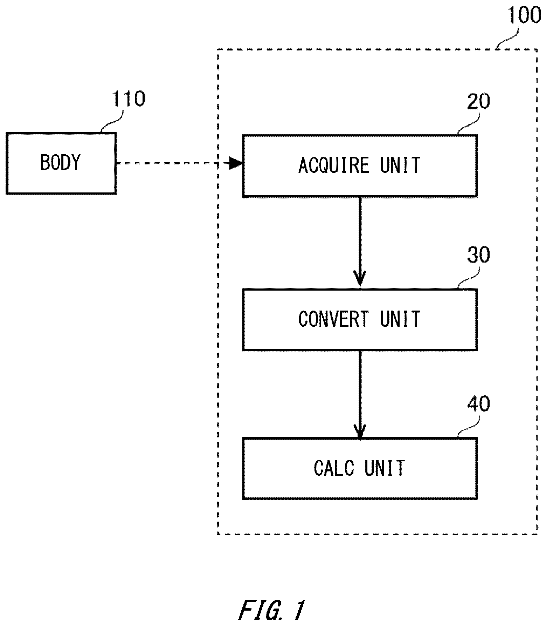

Fig. 1 shows an outline of a configuration of an estimating device 100. The estimating device 100 in the present example includes an acquiring unit 20, a converting unit 30, and a calculating unit 40.

The estimating device 100 estimates blood oxygen concentration of a living body 110. The blood oxygen concentration is oxygen concentration in the blood of the living body 110 and, in one example, refers to oxygen saturation ($SpO_2$). For example, estimating $SpO_2$ can provide an index to determine the respiratory condition of the living body 110. The living body 110 in the present example is, but not limited to, a human being. The living body 110 is requested to be somebody or something that has estimated objects for the estimating device 100 in the blood. For example, the living body 110 may be an animal etc. other than the human being.

$SpO_2$ represents a ratio of oxyhemoglobin $HbO_2$ to reduced hemoglobin Hb in the blood of the living body 110. Oxyhemoglobin $HbO_2$ is hemoglobin that is combined with oxygen, whereas reduced hemoglobin Hb is hemoglobin not combined with oxygen. That is, $SpO_2$ represents how much ratio of hemoglobin is combined with oxygen. The light absorption degrees of oxyhemoglobin $HbO_2$ and reduced hemoglobin Hb are different depending on wavelengths. For example, reduced hemoglobin Hb absorbs light having a wavelength of red more than oxyhemoglobin $HbO_2$. By using this characteristic, $SpO_2$ is calculated.

The acquiring unit 20 acquires information related to the living body 110. In one example, the acquiring unit 20 acquires, as the information related to the living body 110, optical information of the living body 110. The acquiring unit 20 may acquire optical information of the whole body of the living body 110, or optical information of a single part of the living body 110. The optical information is optical information related to the living body 110 that is acquired by a camera or an optical device. The optical information may be an image data such as a still image or a moving image. Also, the acquiring unit 20 acquires pulse wave information from the optical information of the living body 110. The pulse wave information is information related to a pulse wave indicating a time waveform by pulsation of the blood vessel of the living body 110.

The converting unit 30 converts signals included in the optical information acquired by the acquiring unit 20 into color-difference signals. In one example, the converting unit 30 converts a part of signals of the image data into color-difference signals. The converting unit 30 converts the signals included in the optical information into first color-difference signals, and into second color-difference signals that are different from the first color-difference signal. Also, the converting unit 30 may convert the part of the signals of the image data into luminance signals. For example, the converting unit 30 converts the optical information of the living body 110 into luminance signals Y and into color-difference signals Cb, Cr.

The calculating unit 40 calculates feature quantity of the living body 110 from the optical information acquired by the acquiring unit 20. The calculating unit 40 calculates the blood oxygen concentration of the living body 110 based on the first color-difference signal and the second color-difference signal that are converted by the converting unit 30. The calculating unit 40 calculates, using the ratio of the first color-difference signal to the second color-difference signal, the blood oxygen concentration of the living body 110. Also, the calculating unit 40 may calculate the blood oxygen concentration based on the difference between the first color-difference signal and the second color-difference signal. In one example, the calculating unit 40 calculates $SpO_2$ from the Cb signal. For example, the calculating unit 40 is a semiconductor device such as a Large-Scale Integration (LSI).

Here, the feature quantity of the living body 110 is what is obtained by quantifying the feature related to conditions of the living body. In the present specification, the feature quantity of the living body 110 especially refers to the blood oxygen concentration of the living body 110. Note that the feature quantity of the living body 110 may include: feature quantity related to the pulse wave of the living body 110; feature quantity related to the blood pressure of the living body 110; feature quantity related to the age of the living body 110; feature quantity related to activities of the living body 110, or the like.

[Example 1]

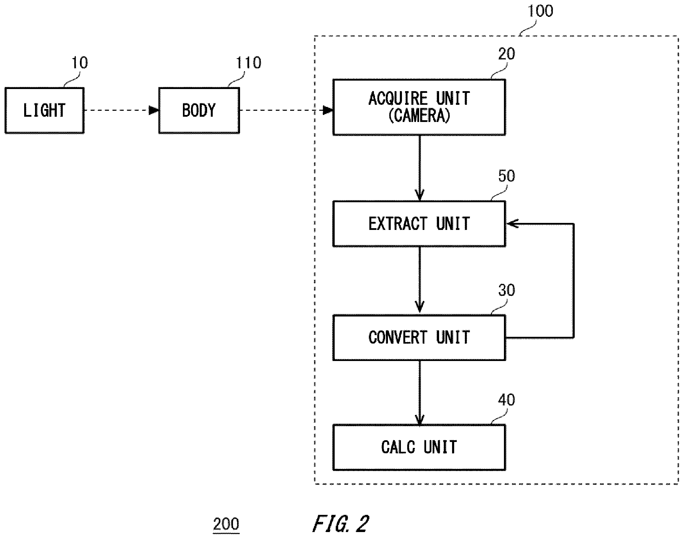

Fig. 2 shows an outline of a configuration of an estimating system 200 according to Example 1. The estimating system 200 includes a light source 10 and the estimating device 100. The estimating device 100 in the present example includes the acquiring unit 20, the converting unit 30, the calculating unit 40, and an extracting unit 50. Note that the estimating device 100 may also include the light source 10.

The light source 10 irradiates the living body 110 with light having a predetermined wavelength. The light emitted from the light source 10 may include a plurality of wavelengths. In one example, the light emitted from the light source 10 includes visible light. Note that the light source 10 may emit light including light having wavelengths other than the wavelengths of visible light. For example, the light source 10 is a white light source. The white light source may be environment light, a fluorescent lamp, a LED bulb, sunlight, or the like.

The acquiring unit 20 has a camera. The acquiring unit 20 in the present example acquires optical information related to the living body 110 by photographing the living body 110 with the camera. For example, the acquiring unit 20 acquires, as the pulse wave information of the living body 110, color-difference signals that are acquired from the optical information of the living body 110. When having a camera, the acquiring unit 20 acquires image information in addition to the pulse wave information from the optical information. For example, the acquiring unit 20 acquires, as the image information, information such as age, sex of the living body 110 that is estimated from the image of the living body 110. Note that the optical information may be image data such as a still image or a moving image.

The extracting unit 50 identifies a region of interest (ROI) of the living body 110 and extracts part of the optical information corresponding to the ROI. The extracting unit 50 identifies the ROI of the living body 110 based on the optical information acquired by the acquiring unit 20 or the signals converted by the converting unit 30. In one example, the extracting unit 50 identifies the ROI of the living body 110 from the image photographed by the acquiring unit 20. Also, the extracting unit 50 may detect the ROI of the living body 110 from the luminance signal converted by the converting unit 30. For example, the extracting unit 50 identifies a position of the face of the living body 110 based on the luminance signals acquired from the optical information, and extracts image data corresponding to the face from the optical information. In this case, the converting unit 30 converts signals of the face image data into color-difference signals. The extracting unit 50 in the present example identifies a position of the nose of the living body 110 based on the optical information, and extracts image data corresponding to the nose of the living body 110 from the optical information. Note that the extracting unit 50 may identify the ROI, not from the luminance signals, but from a gray scale image of the living body 110.

The converting unit 30 converts the optical information extracted by the extracting unit 50 into color-difference signals. The converting unit 30 in the present example converts the signals of the nose image data of the living body 110 into color-difference signals. Thereby, the calculating unit 40 calculates the feature quantity of the living body 110 based on the color-difference signals of the nose of the living body 110.

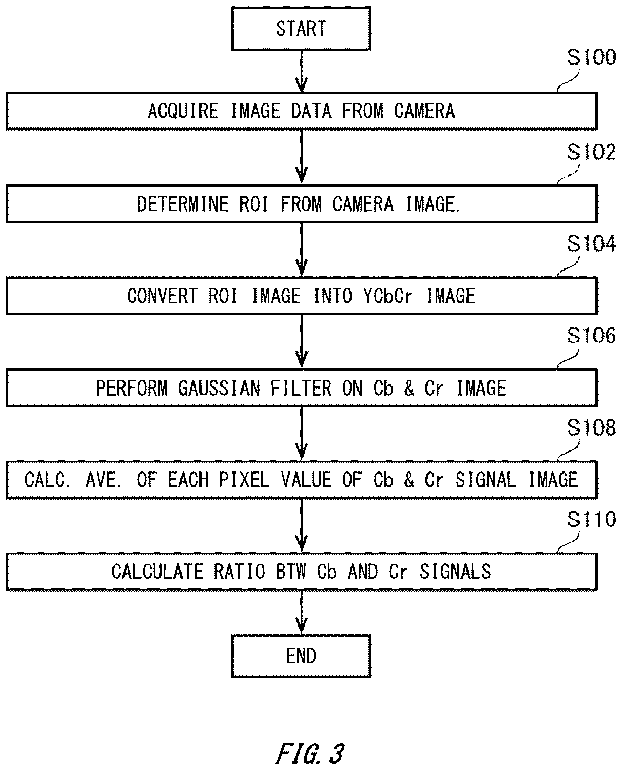

Fig. 3 is an exemplary flowchart illustrating operations of the estimating device 100 according to Example 1.

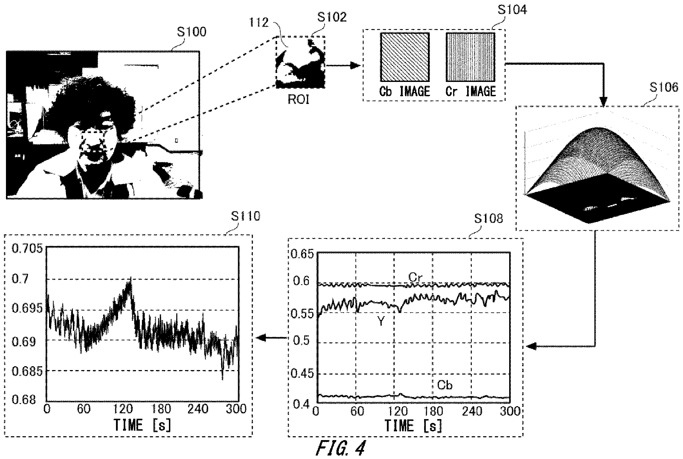

Fig. 4 shows an exemplary outline of an estimating method of the estimating device 100 according to Example 1.

In step S100, the acquiring unit 20 acquires the optical information of the living body 110. The acquiring unit 20 in the present example acquires optical information of a human being who is the living body 110. The acquiring unit 20 has a camera and acquires image data of the living body 110.

In step S102, the extracting unit 50 identifies the ROI of the living body 110 from the optical information.

The extracting unit 50 in the present example identifies the ROI of the living body 110 from the camera image. The extracting unit 50 identifies a region for the nose 112 as the ROI. The acquiring unit 20 extracts a ROI image corresponding to the identified region for the nose 112. The extracting unit 50 in the present example identifies a position of the nose 112 of the living body 110 by image recognition of the camera image of the living body 110. Also, the extracting unit 50 may identify a position of the nose 112 of the living body 110 by detecting the luminance signal.

In step S104, the converting unit 30 performs color conversion of the ROI image. The converting unit 30 in the present example converts the ROI image into YCbCr image. For example, for conversion from the RGB image into YCbCr image, YCbCr signals are expressed by the following formulas. Note that the conversion equation for YCbCr signals in the present example is one example, and not limited to this. The converting unit 30 acquires Cb image and Cr image from the extracted ROI image.

$$Y = 0.30R + 0.59G + 0.11B$$

$$Cb = -0.17R - 0.33G + 0.50B$$

$$Cr = 0.50R - 0.42G - 0.08B$$

In step S106, the calculating unit 40 applies filtering processing to the Cb image and the Cr image. By the filtering processing, the calculating unit 40 smooths the Cb image and the Cr image. The calculating unit 40 in the present example applies Gaussian filter to the Cb image and the Cr image.

The Gaussian distribution in the present example is expressed by the following equation.

$$f(x, y) = \frac{1}{2\pi\sigma^2} \exp\left(-\frac{x^2 + y^2}{2\sigma^2}\right)$$

In step S108, the calculating unit 40 calculates the average of each pixel value of the image of the color-difference signals. The calculating unit 40 in the present example calculates the average of each pixel value of the Cb image and the Cr image. The calculating unit 40 improves estimation accuracy of the blood oxygen concentration, by calculating the average of the Cb image and the Cr image.

In step S110, the calculating unit 40 calculates the ratio of the color-difference signals. Thereby, the calculating unit 40 can estimate the blood oxygen concentration of the living body 110. The calculating unit 40 in the present example calculates the ratio of the Cb signal to the Cr signal. In addition to the ratio of the Cb signal to the Cr signal, the calculating unit 40 may calculate difference between the Cb signal and the Cr signal.

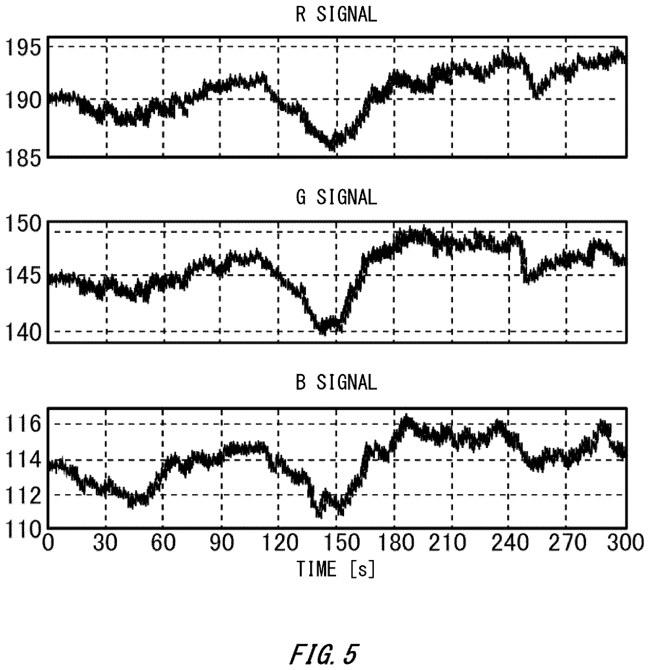

Fig. 5 shows exemplary RGB signals acquired by the acquiring unit 20. The vertical axis represents the signal strength of each RGB signal, and the horizontal axis represents time (sec). In the present example, RGB signals of 0-300 seconds of time are shown.

The signal strengths of the R signal, the G signal, and the B signal have similar waveforms to each other. That is, the waveforms have its ridges or valleys at approximately the same timing. For example, corresponding to decrease of blood volume around 150 seconds, valleys are caused in the waveforms. That is, the signal strengths of the R signal, the G signal, and the B signal are changed corresponding to the blood volume of the living body 110. Thus, if the signal strengths of the R signal, the G signal, and the B signal are only observed and the change corresponding to the blood volume is dominant, it becomes difficult to acquire the change in the blood oxygen concentration. In order to acquire the blood oxygen concentration using the R signal, the G signal, and the B signal, it is necessary to compare signals that are acquired at the timing of the pulse of the living body 110 being constant to keep the influence of the blood volume constant. For example, for estimating the blood oxygen concentration using the R signal, the G signal, and the B signal, it is necessary to compare the signals at the peak and the bottom of the pulse. Thus, for estimation of the blood oxygen concentration by calculations using the RGB signals, it is necessary to use AC component of the pulse wave of the living body 110.

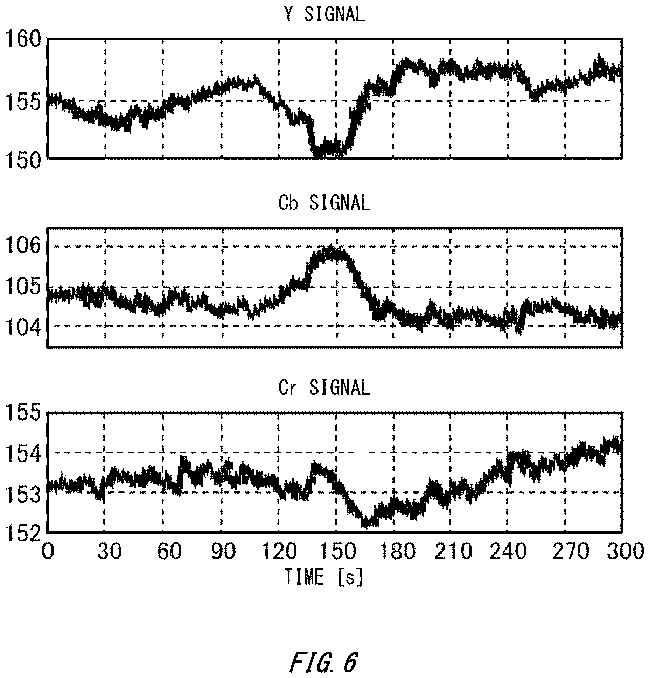

Fig. 6 shows exemplary YCbCr signals acquired by the acquiring unit 20. The vertical axis represents the signal strength of each YCbCr signals, and the horizontal axis represents time (sec). In the present example, YCbCr signals of 0-300 seconds of time are shown.

The signal strengths of the Y signal, the Cb signal, and the Cr signal have waveforms that behave differently from each other. For example, the Y signal has a valley of the signal strength around 150 seconds. On the other hand, the Cb signal has a ridge of the signal strength around 150 seconds. The Cr signal has neither ridge nor valley of the signal strength around 150 seconds. That is, the estimating device 100 can separate the Y signal that is a luminance signal dominantly influenced by the blood volume from the Cb signal and the Cr signal that are color-difference signals not dominantly influenced by the blood volume, and process them separately. Also, the estimating device 100 focuses only on the change of the arterial blood and regards absorption other than absorption by blood as constant. Thereby, by calculations using the Cb signal and the Cr signal, the estimating device 100 can estimate the blood oxygen concentration of the living body 110. Thus, for estimation of the blood oxygen concentration by the calculations using the YCbCr signal, it is not necessary to use the AC component of the pulse wave of the living body 110. Also, the estimating device 100 according to the present specification can estimate the blood oxygen concentration of the living body 110 without using multiple kinds of lights.

If the blood volume of the living body 110 changes, light absorption amounts of the R signal, the G signal, and the B signal change concurrently. Thus, the signal strengths of the RGB signals in Fig. 5 change, having similar tendencies corresponding to the blood volume of the living body 110. This means that brightness (luminance) of the RGB signals change. Thus, the Y signal as the luminance signal in Fig. 6 changes similarly to the RGB signals and becomes a signal dominantly influenced by the blood volume. On the other hand, if the blood oxygen saturation changes, the light absorption amount shows different changes depending on the light wavelengths, which gives a dominant influence on the Cb signal and the Cr signal. In this manner, in the estimating device 100, the Cb signal and the Cr signal are dominantly influenced not by the blood volume, but by the blood oxygen saturation, and thus the blood oxygen concentration can be estimated not using the AC component of the pulse wave of the living body 110, but using the bias component of at least one of the Cb signal and the Cr signal. On the other hand, when using the RGB signals that is dominantly influenced by the blood volume, the influence by the blood volume cannot be removed from the bias component of the RGB signals, and thus it is difficult to estimate the blood oxygen concentration from the bias component of the RGB signals.

As described above, by using the YCbCr signals, the estimating device 100 can estimate the blood oxygen concentration without using the AC component of the pulse wave of the living body 110. In other words, the estimating device 100 can estimate the blood oxygen concentration without using the ratio of the peak to the bottom of the pulse wave of the living body 110.

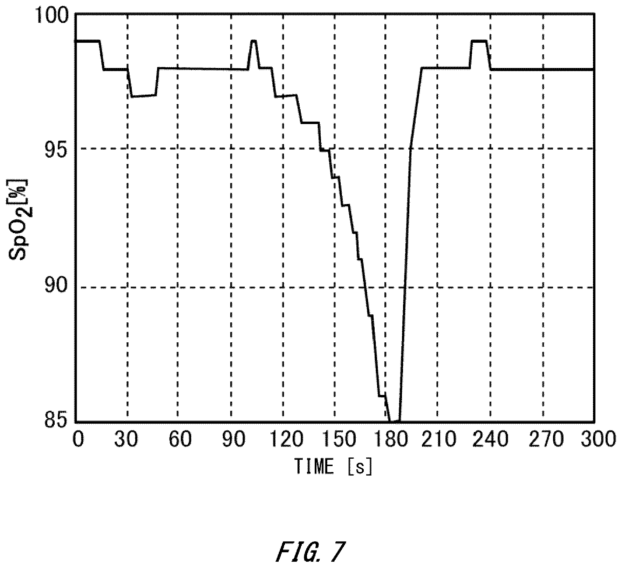

Fig. 7 shows an actual value of $SpO_2$ that is detected from the living body 110. The vertical axis represents $SpO_2$ (%), and the horizontal axis represents time (sec).

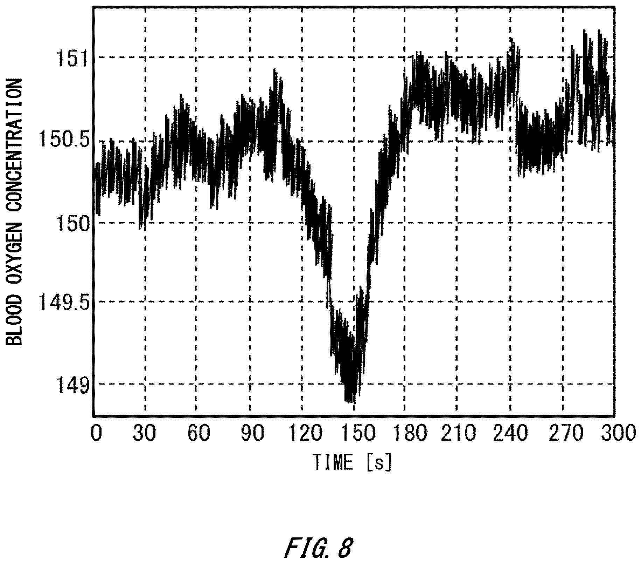

Fig. 8 shows an estimated value of the blood oxygen concentration that is estimated by the estimating device 100. The horizontal axis represents time (sec). In the present example, the blood oxygen concentration of 0-300 seconds is estimated.

The actual value of $SpO_2$ in Fig. 7 is measured using a pulse oximeter. The pulse oximeter calculates the blood oxygen concentration of the living body 110 using light having wavelengths of red and infrared.

The blood oxygen concentration in Fig. 8 is estimated from the optical information of the living body 110 that is acquired by the estimating device 100, using the color-difference signal.

When Fig. 7 and Fig. 8 are compared, it can be seen that the estimating device 100 estimates blood oxygen concentration having a closer waveform to that of the actual value. Note that there is a valley of $SpO_2$ around 180 seconds in Fig. 7, whereas there is a valley of the blood oxygen concentration around 150 seconds in Fig. 8. Such difference in the waveforms between Fig. 7 and Fig. 8 is due to time difference caused by the signal processing by the pulse oximeter in Fig. 7. Accordingly, it can be seen that the estimating device 100 can estimate, as similar to the actual value measured by the pulse oximeter, the change in the waveform of the blood oxygen concentration.

Note that, the value in the vertical axis in Fig. 8 is not the same as the $SpO_2$ value in Fig. 7. However, as similar to what other $SpO_2$ measurement instruments do, checking in advance the correspondence relation between the values output from the estimating device 100 and the actual values of $SpO_2$ allows a value corresponding to $SpO_2$ value to be output.

[Example 2]

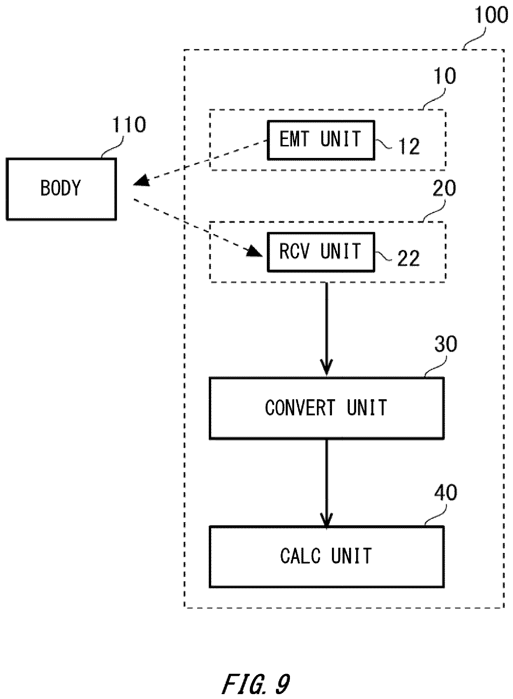

Fig. 9 shows an exemplary configuration of the estimating device 100 according to Example 2. The estimating device 100 in the present example includes a light-emitting unit 12 as the light source 10. Also, the estimating device 100 includes a light-receiving unit 22 as the acquiring unit 20.

The estimating device 100 acquires optical information, based on reflected light from or transmitted light through the living body 110. The estimating device 100 acquires the optical information of the living body 110 by detecting light corresponding to the light emitted by the light-emitting unit 12 by the light-receiving unit 22. In one example, the estimating device 100 is provided with wearable terminals.

The light-emitting unit 12 has a light emitting diode (LED) to irradiate the living body 110 with light having a predetermined wavelength. The light-emitting unit 12 may have a plurality of LEDs that irradiate the living body 110 with lights having respective individual wavelengths. The light-emitting unit 12 irradiates the living body 110 with lights having a first wavelength $\lambda_1$, and a second wavelength $\lambda_2$ that is different from the first wavelength $\lambda_1$. In one example, the light-emitting unit 12 irradiates the living body 110 with lights having a plurality of wavelengths in a visible light region. Also, the light-emitting unit 12 may irradiate the living body 110 with lights including wavelengths outside the visible light region.

Also, the light-emitting unit 12 irradiates lights the living body 110 having the first wavelength $\lambda_1$ and the second wavelength $\lambda_2$. In one example, the light-emitting unit 12 emits light having, as the first wavelength $\lambda_1$, a wavelength where absorption coefficient of reduced hemoglobin Hb is greater than that of oxyhemoglobin $HbO_2$ in the blood of the living body 110. Also, the light-emitting unit 12 emits light having, as the second wavelength $\lambda_2$, a wavelength where absorption coefficient of reduced hemoglobin Hb is smaller than that of oxyhemoglobin $HbO_2$ in the blood of the living body 110. For example, the light-emitting unit 12 emits lights having wavelengths of blue and red. The light-emitting unit 12 may irradiate the living body 110 with lights having both of the first wavelength $\lambda_1$ and the second wavelength $\lambda_2$. Also, the light-emitting unit 12 may irradiate the living body 110 with lights having the first wavelength $\lambda_1$, the second wavelength $\lambda_2$, and a third wavelength $\lambda_3$. In one example, the light-emitting unit 12 emits light having, as the third wavelength $\lambda_3$, a wavelength of green between the first wavelength $\lambda_1$ and the second wavelength $\lambda_2$. For example, the light-emitting unit 12 irradiates the living body 110 with light of white.

Note that the light-emitting unit 12 concurrently irradiates the living body 110 with lights having a plurality of wavelengths. Also, the light-emitting unit 12 may sequentially irradiate lights the living body 110 with having a plurality of wavelengths. In one example, the light-emitting unit 12 may include a filtering unit to remove part of light having a certain wavelength region from the irradiation light.

The light-receiving unit 22 is an optical device to detect reflected light or transmitted light of the light emitted from the light-emitting unit 12. The light-receiving unit 22 may have an imaging device such as a CCD image sensor and a CMOS image sensor. The light-receiving unit 22 in the present example receives reflected light that is obtained by the irradiation light reflected from the living body 110. The light-receiving unit 22 may receive reflected lights based on irradiation lights having a plurality of wavelengths that are concurrently emitted from the light-emitting unit 12 to the living body 110. Also, the light-receiving unit 22 may detect transmitted light obtained by the infrared light (IR) emitted to the skin of the living body 110 by the light-emitting unit 12 transmitting through the living body 110. Thereby, the acquiring unit 20 acquires the optical information of the living body 110.

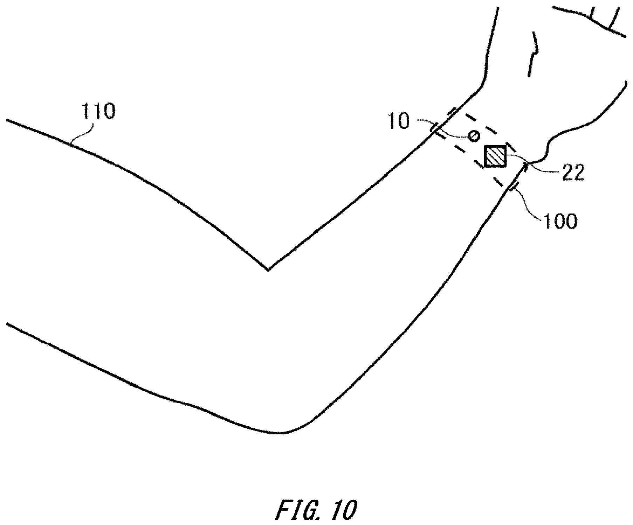

Fig. 10 shows an exemplary configuration of the estimating device 100 according to Example 2. The estimating device 100 is a wearable device including the light source 10 and the light-receiving unit 22. The estimating device 100 in the present example is worn on the wrist of the living body 110.

Also, the light-emitting unit 12 irradiates the living body 110 with lights having the first wavelength $\lambda_1$ and the second wavelength $\lambda_2$. In one example, the light-emitting unit 12 emits light having, as the first wavelength $\lambda_1$, a wavelength where absorption coefficient of reduced hemoglobin Hb is greater than that of oxyhemoglobin $HbO_2$ in the blood of the living body 110. Also, the light-emitting unit 12 emits light having, as the second wavelength $\lambda_2$, a wavelength where absorption coefficient of reduced hemoglobin Hb is smaller than that of oxyhemoglobin $HbO_2$ in the blood of the living body 110. For example, the light-emitting unit 12 emits lights having wavelengths of blue and red. The light-emitting unit 12 may irradiate the living body 110 with lights having both of the first wavelength $\lambda_1$ and the second wavelength $\lambda_2$. Also, the light-emitting unit 12 may irradiate the living body 110 with lights having the first wavelength $\lambda_1$, the second wavelength $\lambda_2$, and a third wavelength $\lambda_3$. In one example, the light-emitting unit 12 emits light having, as the third wavelength $\lambda_3$, a wavelength of green between the first wavelength $\lambda_1$ and the second wavelength $\lambda_2$. For example, the light-emitting unit 12 irradiates the living body 110 with light of white.

The light-receiving unit 22 detects reflected light from or transmitted light through the living body 110 of the light emitted from the light source 10. The light-receiving unit 22 in the present example has a light receiving element to receive the reflected light from the living body 110. The estimating device 100 in the present example includes an extracting unit 50 and processes an output of the light-receiving unit 22 as the ROI.

The estimating device 100 in the present example can easily estimate the blood oxygen concentration of the living body 110 by being worn on the wrist of the living body 110. Thereby, the estimating device 100 can easily estimate the blood oxygen concentration over a long time. Also, the estimating device 100, by including an acceleration sensor, may measure an activity amount etc. of the living body 110, in addition to the blood oxygen concentration. In this case, the estimating device 100 can acquire information combining the blood oxygen concentration and the feature quantity of the living body 110.

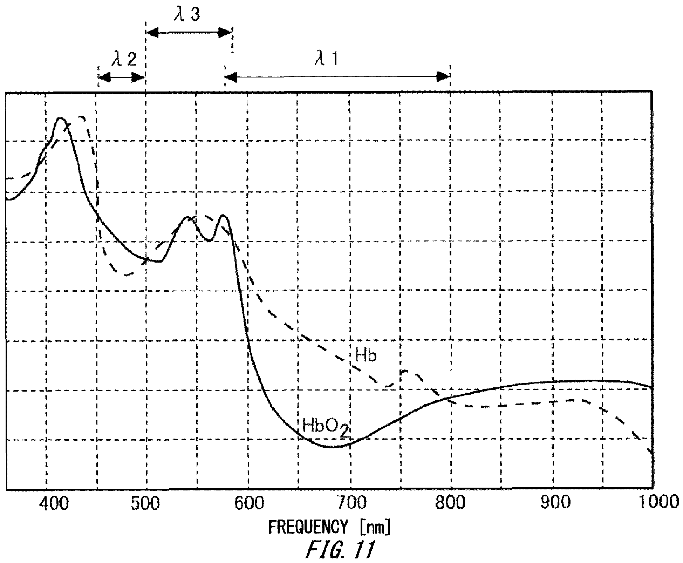

Fig. 11 shows exemplary wavelengths of irradiation lights when the lights emitted from the light source 10 include wavelengths of visible light. The light source 10 in the present example emits lights having three wavelengths: the first wavelength $\lambda_1$, the second wavelength $\lambda_2$, and the third wavelength $\lambda_3$.

The light source 10 emits light having the first wavelength $\lambda_1$ where absorption coefficient of reduced hemoglobin Hb is greater than that of oxyhemoglobin $HbO_2$ in the blood of the living body 110. The light source 10 in the present example selects, as the first wavelength $\lambda_1$, a wavelength of 575-800 nm. Also, the light source 10 emits light having the second wavelength $\lambda_2$ where absorption coefficient of reduced hemoglobin Hb is smaller than that of oxyhemoglobin $HbO_2$ in the blood of the living body 110. The light source 10 in the present example selects, as the second wavelength $\lambda_2$, a wavelength of 450-500 nm. Also, the light source 10 emits light of the third wavelength $\lambda_3$ between the first wavelength $\lambda_1$ and the second wavelength $\lambda_2$. The light source 10 in the present example selects, as the third wavelength $\lambda_3$, a wavelength of 500-580 nm.

As described above, the estimating device 100 in the present example, even using irradiation light including only a wavelength of visible light, can select a wavelength having different absorption coefficients between oxyhemoglobin $HbO_2$ and reduced hemoglobin Hb. The estimating device 100 can estimate the blood oxygen concentration of the living body 110, if a wavelength having different absorption coefficients between oxyhemoglobin $HbO_2$ and reduced hemoglobin Hb can be selected. In this manner, the irradiation light of the estimating device 100 may have only a wavelength of visible light.

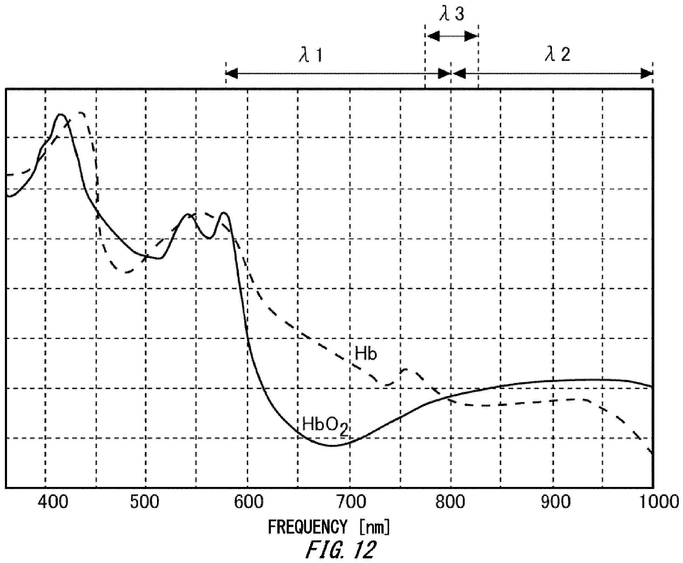

Fig. 12 shows exemplary wavelengths of the irradiation lights when the lights emitted from the light source 10 include wavelengths of other than visible light. The light source 10 in the present example emits lights having three wavelengths: the first wavelength $\lambda_1$, the second wavelength $\lambda_2$, and the third wavelength $\lambda_3$.

The light source 10 emits light having the first wavelength $\lambda_1$ where absorption coefficient of reduced hemoglobin Hb is greater than that of oxyhemoglobin $HbO_2$ in the blood of the living body 110. The light source 10 in the present example selects, as the first wavelength $\lambda_1$, a wavelength of 575-800 nm. Also, the light source 10 emits light having the second wavelength $\lambda_2$ where absorption coefficient of reduced hemoglobin Hb is smaller than that of oxyhemoglobin $HbO_2$ in the blood of the living body 110. The light source 10 in the present example selects, as the second wavelength $\lambda_2$, a wavelength of 800-1000 nm. Also, the light source 10 emits light of the third wavelength $\lambda_3$ between the first wavelength $\lambda_1$ and the second wavelength $\lambda_2$. The light source 10 in the present example selects, as the third wavelength $\lambda_3$, a wavelength of 775-825 nm.

As described above, even using irradiation light including wavelengths other than the wavelengths of visible light in addition to the wavelengths of visible light, the estimating device 100 in the present example can select a wavelength having different absorption coefficients between oxyhemoglobin $HbO_2$ and reduced hemoglobin Hb. The estimating device 100 can estimate the blood oxygen concentration of the living body 110, if a wavelength having different absorption coefficients between oxyhemoglobin $HbO_2$ and reduced hemoglobin Hb can be selected. In this manner, the irradiation light of the estimating device 100 may have a wavelength other than the wavelength of visible light.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

EXPLANATION OF REFERENCES

10: light source; 12: light-emitting unit; 20: acquiring unit; 22: light-receiving unit; 30: converting unit; 40: calculating unit; 50: extracting unit; 100: estimating device; 110: living body; 112: nose; 200: estimating system

**Claims**

1. An estimating device to estimate blood oxygen concentration of a living body, the estimating device comprising:

    an acquiring unit to acquire optical information from the living body;
    a converting unit to convert a signal included in the optical information into a first color-difference signal; and
    a calculating unit to calculate blood oxygen concentration of the living body based on the first color-difference signal.

**2.** The estimating device according to claim 1, wherein the acquiring unit acquires the optical information based on reflected light from the living body.

**3.** The estimating device according to claim 1 or 2, wherein

the converting unit converts a signal included in the optical information into a second color-difference signal that is different from the first color-difference signal, and
the calculating unit calculates the blood oxygen concentration based on the first color-difference signal and the second color-difference signal.

**4.** The estimating device according to claim 3, wherein the calculating unit calculates the blood oxygen concentration based on a ratio of the first color-difference signal to the second color-difference signal.

**5.** The estimating device according to claim 3 or 4, wherein
the calculating unit calculates the blood oxygen concentration based on difference between the first color-difference signal and the second color-difference signal.

**6.** The estimating device according to any one of claims 1 to 5, further comprising an extracting unit to identify a position of a face of the living body based on the optical information and extract image data corresponding to the face from the optical information, wherein
the converting unit converts a signal of image data of the face into the color-difference signal.

**7.** The estimating device according to claim 6, further comprising an extracting unit to identify a position of a face of the living body based on a luminance signal acquired from the optical information and extract image data corresponding to the face from the optical information, wherein
the converting unit converts a signal of image data of the face into the color-difference signal.

**8.** The estimating device according to any one of claims 1 to 7, further comprising an extracting unit to identify a nose of the living body based on the optical information and extract image data corresponding to the nose from the optical information, wherein
the converting unit converts a signal of image data of the nose into the color-difference signal.

**9.** The estimating device according to claim 7, further comprising an extracting unit to identify a nose of the living body based on a luminance signal acquired by the optical information and extract image data corresponding to the nose from the optical information, wherein
the converting unit converts a signal of image data of the nose into the color-difference signal.

**10.** The estimating device according to any one of claims 1 to 9, further comprising
a light source to irradiate the living body with light having a predetermined wavelength.

**11.** The estimating device according to claim 10, wherein the light source emits the lights including a first wavelength, and a second wavelength that is different from the first wavelength.

**12.** The estimating device according to claim 11, wherein the light source emits light having, as the first wavelength, a wavelength of 575 to 800 nm, at the wavelength absorption coefficient of reduced hemoglobin being greater than absorption coefficient of oxyhemoglobin in blood of the living body, and emits light having, as the second wavelength, at the wavelength absorption coefficient of reduced hemoglobin is smaller than absorption coefficient of oxyhemoglobin in blood of the living body.

**13.** The estimating device according to claim 11 or 12, wherein
the light source emits the lights including a third wavelength that is different from the first wavelength and the second wavelength.

**14.** The estimating device according to claim 13, wherein the third wavelength includes light having a wavelength between the first wavelength and the second wavelength.

**15.** The estimating device according to any one of claims 11 to 14, wherein
the light source concurrently irradiates the living body with lights having the first wavelength and the second wavelength.

**16.** The estimating device according to claim 13 or 14, wherein
the light source concurrently irradiates the living body with lights having the first wavelength, the second wavelength, and the third wavelength.

**17.** The estimating device according to any one of claims 10 to 16, wherein
the light source irradiates the living body with the light of white.

**18.** The estimating device according to any one of claims 1 to 17, wherein
the acquiring unit has a camera, and acquires the optical information by photographing the living body.

*FIG. 1*

100

10

110

20

LIGHT - - - ► BODY - - - ► ACQUIRE UNIT
(CAMERA)

50

EXTRACT UNIT

30

CONVERT UNIT

40

CALC UNIT

200    *FIG. 2*

```
                        ┌─────────────┐
                        │    START    │
                        └──────┬──────┘
                               │                        S100
                               ▼
        ┌──────────────────────────────────────────────────┐
        │       ACQUIRE  IMAGE  DATA  FROM  CAMERA           │
        └──────────────────────┬───────────────────────────┘
                               │                        S102
                               ▼
        ┌──────────────────────────────────────────────────┐
        │       DETERMINE  ROI  FROM  CAMERA  IMAGE.         │
        └──────────────────────┬───────────────────────────┘
                               │                        S104
                               ▼
        ┌──────────────────────────────────────────────────┐
        │       CONVERT  ROI  IMAGE  INTO  YCbCr  IMAGE      │
        └──────────────────────┬───────────────────────────┘
                               │                        S106
                               ▼
        ┌──────────────────────────────────────────────────┐
        │     PERFORM  GAUSSIAN  FILTER  ON  Cb & Cr  IMAGE  │
        └──────────────────────┬───────────────────────────┘
                               │                        S108
                               ▼
        ┌──────────────────────────────────────────────────┐
        │ CALC.  AVE.  OF EACH PIXEL VALUE OF Cb & Cr SIGNAL IMAGE │
        └──────────────────────┬───────────────────────────┘
                               │                        S110
                               ▼
        ┌──────────────────────────────────────────────────┐
        │     CALCULATE  RATIO  BTW  Cb  AND  Cr  SIGNALS    │
        └──────────────────────┬───────────────────────────┘
                               │
                               ▼
                        ┌─────────────┐
                        │     END     │
                        └─────────────┘
```

*FIG. 3*

*FIG. 4*

FIG. 5

FIG. 6

*FIG. 7*

*FIG. 8*

*FIG. 9*

FIG. 10

FIG. 11

FIG. 12

## INTERNATIONAL SEARCH REPORT

| International application No. |
|---|
| PCT/JP2017/015908 |

**A. CLASSIFICATION OF SUBJECT MATTER**
*A61B5/1455*(2006.01)i, *A61B5/00*(2006.01)i

According to International Patent Classification (IPC) or to both national classification and IPC

**B. FIELDS SEARCHED**

Minimum documentation searched (classification system followed by classification symbols)
A61B5/1455-5/1464, A61B5/00, A61B1/00-1/32

Documentation searched other than minimum documentation to the extent that such documents are included in the fields searched
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2014-529439 A (Koninklijke Philips N.V.), 13 November 2014 (13.11.2014), claims 1, 7; paragraphs [0073] to [0144]; fig. 1 to 10 & US 2014/0206965 A1 claims 1, 7; paragraphs [0092] to [0163]; fig. 1 to 10 & WO 2013/030739 A1 & EP 2748762 A1 & CN 103765436 A | 1-18 |
| Y | JP 2013-150772 A (Sony Corp.), 08 August 2013 (08.08.2013), paragraphs [0051] to [0058]; fig. 11 & WO 2013/099509 A1 | 1-18 |

| ☒ Further documents are listed in the continuation of Box C. | ☐ See patent family annex. |
|---|---|

| \* | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 July 2017 (07.07.17) | 18 July 2017 (18.07.17) |

| Name and mailing address of the ISA/ Japan Patent Office 3-4-3,Kasumigaseki,Chiyoda-ku, Tokyo 100-8915,Japan | Authorized officer Telephone No. |
|---|---|

Form PCT/ISA/210 (second sheet) (January 2015)

**INTERNATIONAL SEARCH REPORT**

| International application No. |
| --- |
| PCT/JP2017/015908 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2010-233908 A (Konica Minolta Sensing, Inc.), 21 October 2010 (21.10.2010), paragraphs [0002] to [0005] (Family: none) | 1-18 |
| A | WO 2013/035694 A1 (Fujifilm Corp.), 14 March 2013 (14.03.2013), paragraphs [0034] to [0056]; fig. 6A to 12 & US 2014/0155717 A1 paragraphs [0067] to [0089]; fig. 6A to 12 & EP 2754381 A1 & CN 103732116 A | 1-18 |
| A | JP 2007-54483 A (Olympus Corp.), 08 March 2007 (08.03.2007), paragraph [0077]; fig. 9 (Family: none) | 1-18 |
| A | US 2016/0106352 A1 (PEETERS,W.H.), 21 April 2016 (21.04.2016), paragraphs [0004], [0030]; fig. 1 & WO 2016/059528 A1 & CN 106793956 A | 1-18 |

Form PCT/ISA/210 (continuation of second sheet) (January 2015)

**EP 3 449 825 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012143399 A
- JP 2012125501 A
- JP H6285050 B
- JP 2014529439 W

**Non-patent literature cited in the description**

- Non-contact estimation of heart rate and oxygen saturation using ambient light. **UFUK BAL.** Biomedical Optics Express 86. The Optical Society of America, 01 January 2015, vol. 6, 86-97